# EUROPEAN PATENT APPLICATION

(11) **EP 4 389 137 A1**
(43) Date of publication of application: **26.06.2024**
(21) Application number: 22383279.1
(22) Date of filing: 23.12.2022
(51) Int. Cl.: A61K 36/87, A23L 33/105, A61K 31/216, A61K 31/352, A61P 25/28

(54) **POLYPHENOLIC EXTRACTS FROM GRAPEVINE LEAVES**

(71) Applicant: Fundación Tecnalia Research & Innovation, 48160 Derio - Bizkaia (ES); Universidad del País Vasco/Euskal Herriko Unibertsitatea, 48940 Leoia, Bikaia (ES)
(72) Inventor: LÓPEZ DE ARMENTIA VICANDI, Iratxe, 48160 DERIO - BIZKAIA (ES); MATUTE ALMAU, Carlos, 48940 LEIOA (ES); ELEJALDE CARAVACA, Edurne, 48160 DERIO - BIZKAIA (ES); CAPETILLO GONZÁLEZ DE ZÁRATE, Estibaliz, 48940 LEIOA (ES); ALBERDI ALFONSO, Elena, 48940 LEIOA (ES)
(74) Representative: ZBM Patents - Zea, Barlocci & Markvardsen

(57) **Abstract**

It is provided a *Vitis vinifera* leaf extract comprising caftaric acid, a quercetin derivative, and a kaempferol derivative, characterized in that the quercetin derivative/kaempferol derivative weight ratio is from 4.2 to 5.0; a pharmaceutical composition comprising it; and the extract for use in the prevention or in the treatment of a neurodegenerative disease. It is also provided a process for the preparation of the *Vitis vinifera* leaf extract by ultrasound assisted extraction (UAE).

## Description

### Technical Field

The present invention belongs to the field of grapevine extracts. In particular, it relates to a grapevine leaves extract, a process for their preparation, a pharmaceutical composition containing it, and to the extract for use for the prevention or treatment of neurodegenerative diseases.

### Background Art

Alzheimer's disease is a progressive neurodegenerative disease characterized by memory loss and cognitive impairment. Several mechanisms have been identified as being involved in neuronal death such as oxidative stress, altered calcium homeostasis, inflammation, metabolic alterations mainly due to the accumulation of amyloid peptide and Tau protein. These mechanisms lead to a loss of connections between neurons, which ultimately results in programmed cell death. It is therefore a neurodegenerative pathology characterized by a complex and progressive cognitive failure and which, to date, has no effective pharmacological treatment.

The most widely accepted pathophysiological model of the disease proposes that amyloid peptide (Aβ) is responsible for cognitive impairment and actively contributes to the development of the disease. As an instance, in Alzheimer's disease, the amyloid plaque as well as Tau tangles in the brain appear long before cognitive symptoms develop. Some work shows that Aβ is directly involved in altering the biology of the synapse, since if its production is blocked by inhibitors of an enzyme necessary for its production, synaptic damage is not observed. This enzyme is involved in many other cellular mechanisms, so inhibiting it is not a viable treatment.

The leaves of *Vitis vinifera* have been used for a long time for the treatment of many disorders or diseases. Particularly, grape polyphenols have been shown to act against oxidative stress and to reduce Aβ peptide formation and aggregation, both responsible, among other, for neurodegenerative diseases such as Alzheimer's disease.

However, there is still a need for more effective therapies against neurodegenerative diseases, particularly that can prevent the developments of the disease in asymptomatic stages.

### Summary of Invention

The inventors have found that by carrying out an extraction from grapevine (*Vitis vinifera*) leaves in specific conditions, an extract rich in polyphenols with a particular weight relation of certain polyphenols is obtained. Particularly, the extract comprises caftaric acid, quercetin derivatives, and kaempferol derivatives, wherein quercetin derivatives, and kaempferol derivatives are in a specific range of weight ratios.

Surprisingly, the grapevine leaf extract of the present invention provides an unexpectedly high neuroprotective effects in experimental models of Alzheimer's diseasecompared with similar extracts of the prior art. Particularly, the extract significantly reduces the oxidative stress induced by oligomers of the amyloid β1-42 (oAβ) peptide in primary cultures of cortical neurons, which is a model of the Alzheimer's disease. Remarkably, said effect was not observed when an extract from the same grapevine was obtained by another extraction methods known in the prior art.

Thus, a first aspect of the present invention relates to a *Vitis vinifera* leaf extract comprising caftaric acid, a quercetin derivative, and a kaempferol derivative, characterized in that the quercetin derivative/kaempferol derivative weight ratio is from 4.2 to 5.0.

Another aspect relates to a pharmaceutical composition comprising a therapeutically effective amount of the *Vitis vinifera* leaf extract defined herein and at least one pharmaceutically acceptable excipient or carrier.

Another aspect relates to the *Vitis vinifera* leaf extract or the pharmaceutical composition defined herein for use in medicine, particularly, for use in the prevention and/or in the treatment of a neurodegenerative disease. This aspect may also be formulated as the use of the *Vitis vinifera* leaf extract or the pharmaceutical composition defined herein for the preparation of a medicament for the prevention and/or the treatment of a neurodegenerative disease. The present disclosure also relates to a method for the prevention or the treatment of a neurodegenerative disease, the method comprising administering a therapeutically effective amount of the *Vitis vinifera* leaf extract or the pharmaceutical composition defined herein, together with pharmaceutically acceptable excipients or carriers, in a subject in need thereof, including a human.

Another aspect of the invention relates to a process for the preparation of the *Vitis vinifera* leaf extract as defined in any one of claims 1 to 3, the process comprising:
a) freeze-drying *Vitis vinifera* leaves,
b) grinding and sieving the freeze-dried leaves in order to obtain a powder of particles having a size of less than 500 µm;
c) carrying out an ultrasound assisted extraction with water in order to obtain an aqueous extracted solution;
d) collecting and filtering the aqueous extracted solution; and
e) optionally, removing water from the aqueous extracted solution in order to obtain a dry extract.

### Brief Description of Drawings

Fig. 1 shows the chromatogram by HPLC/DAD (356 nm) of grape leaf extracts obtained by the Ultrasound Assisted Extraction (UAE) extraction method of Example 1. CA: Caftaric acid; QD: Quercetin derivatives; KD: Kaempferol derivatives.
Fig. 2 shows the significant reduction of the production of free radicals (%) (reactive oxygen species, ROS) in the presence of two extracts (H1 US and H2 US) obtained by Ultrasound Assisted Extraction (UAE) and oligomers of the amyloid β1-42 (oAβ) peptides compared to oAβ alone. Each bar represents the mean +/- standard error of the mean (SEM) (N =4-5; *p<0.05, **p<0.01; Student's t test).
Fig. 3 shows the amount of free radicals (ROS) (%) produced in the presence of an extract (H3 A) obtained by Accelerated Solvent Extraction (ASE) and oAβ (N=5; ns, non-significant; Student's t test). N: number of experiments.
Fig. 4 shows the average particles size distribution for dried grapevine leaves after grinding them into fine powder using a M 20 Universal mill (IKA) and then subjecting the powder to a sieve analyzer as disclosed in the description.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

As used herein, "grapevine leaves extract" or simply "extract" refers to concentrated preparations of grapevine leaves obtained by isolating the active constituents including polyphenols, from the plant by suitable means, particularly by extraction with water.

The term "weight ratio" (weight/weight) refers to the relation of weights of the molecules or compounds indicated. As used herein, "wt%" of a compound refers to the amount of the single compound relative to the total weight of the composition or, if specifically mentioned, of other compound or compound. In the present invention the weight of an extract refers to the dry extract weight or mass.

As mentioned above, a first aspect of the present invention relates to a *Vitis vinifera* leaf extract comprising caftaric acid, quercetin derivatives, and kaempferol derivatives, characterized in that the quercetin derivatives/kaempferol derivatives ratio is from 4.2 to 5.0.

In an embodiment of the *Vitis vinifera* leaf extract, caftaric acid is in an amount from 30 wt% to 45 wt%, particularly from 34 wt% to 43 wt%, with respect to the total amount of caftaric acid, quercetin derivative, and kaempferol derivative.

In another embodiment of the combination of the invention, optionally in combination with any of the embodiments provided above, the extract is a dry extract and the amount of caftaric acid is from 38 wt% to 46 wt%, particularly 40 wt% to 44 wt%, with respect to the total amount of dry extract.

In another embodiment of the combination of the invention, optionally in combination with any of the embodiments provided above, quercetin derivatives are selected from the group consisting of quercetin 3-O-rutinoside (rutin; CAS: 153-18-4), quercetin 3-O-glucoside (isoquercetin; CAS: 482-35-9), quercetin 3-O-galactoside (hyperoside; CAS: 482-36-0), quercetin 3-O-glucuronide (miquelianin; CAS: 22688-79-5), and mixtures thereof; and the kaempferol derivatives are selected from the group consisting of kaempferol 3-O-galactoside (trifolin; CAS: 23627-87-4), kaempferol 3-O-glucoside (astragalin; CAS: 480-10-4), kaempferol 3-O-glucuronide (CAS: 22688-78-4), and mixtures thereof.

Caftaric acid (CAS: 67879-58-7) and all the above-mentioned quercetin derivatives and kaempferol derivatives are commercially available.

In a more particular embodiment, the quercetin derivative is a mixture of 3-O-rutinoside, quercetin 3-O-glucoside, quercetin 3-O-galactoside, and quercetin 3-O-glucuronide; and the kaempferol derivative is a mixture of kaempferol 3-O-galactoside, kaempferol 3-O-glucoside, and kaempferol 3-O-glucuronide.

In another embodiment of the combination of the invention, optionally in combination with any of the embodiments provided above, the weight ratio of quercetin derivatives/kaempferol derivatives weight ratio is 4.2, 4.3, 4.4, 4.5, 4.6, 4.7, 4.8, 4.9, or 5.0.

*Vitis vinifera* is the common grapevine. The extracts of the present invention are obtained from leaves of *Vitis vinifera.*

In an embodiment of the present invention, optionally in combination with any of the embodiments provided above, the *Vitis vinifera* leaf extract is an aqueous extract.

As used herein, the term "aqueous extract" refers to an extract where the extraction has been performed using water as the only solvent.

In another embodiment of the combination of the invention, optionally in combination with any of the embodiments provided above, the extract is a dry extract.

As mentioned above, the invention provides in a second embodiment a pharmaceutical composition comprising a therapeutically effective amount of the *Vitis vinifera* leaf extract defined herein and at least one pharmaceutically acceptable excipient or carrier.

The term "pharmaceutical composition" refers to a composition used for improving or maintaining the health of a subject and comprise pharmaceutically acceptable excipients or carriers.

The expression " therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions, or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

In another embodiment, optionally in combination with any of the embodiments provided above, the pharmaceutical composition comprises the *Vitis vinifera* leaf extract of the invention in an amount from 0.75 µg to 3.0 µg of dry extract, more in particular from 1.0 µg to 2.0 µg.

The pharmaceutical composition of the invention can additionally incorporate other active ingredients which reinforce the beneficial effects of the combination of the invention.

The compositions of the invention can additionally incorporate other active pharmaceutically active agent which reinforce the beneficial effects of the *Vitis vinifera* leaf extract of the invention.

As used herein, the term "pharmaceutically active agent" refers to and agent that has pharmacological activity and is used for curing, mitigating, treating or preventing a disease in a subject, in particular a human, more particularly for the prevention or treatment of a neurodegenerative disease such as Alzheimer's disease.

The pharmaceutically compositions of the invention can be formulated in several forms that include, but are not limited to, solutions, tablets, capsules, granules, suspensions, dispersions, powders, lozenge, chewable candy, candy bar, concentrate, drops, elixir, emulsion, film, gel, granule, chewing gum, jelly, oil, paste, pastille, pellet, soap, sponge, suppository, syrup, chewable gelatin form, or chewable tablet.

The pharmaceutical compositions of the present invention can be prepared according to methods well known in the state of the art. The appropriate excipients and/or carriers, and their amounts, can readily be determined by those skilled in the art according to the type of formulation being prepared. As an instance, excipients or carriers employed for oral administration include, without being limited to, fillers, binders, disintegrates, lubricants, anticaking, glidants or their mixtures.

As mentioned before, the invention provides in a third aspect the extract according to for use in the prevention or in the treatment of a neurodegenerative disease. As shown in the examples, the *Vitis vinifera* leaf extract of the invention significantly reduces the oxidative stress in neurons, and as such it is useful, in particular, in the prevention or in the treatment of the Alzheimer's disease.

As mentioned above, another aspect of the invention relates to a process for the preparation of a *Vitis vinifera* leaf extract as defined in claims 1 or 2, the process comprising a) freeze-drying Vitis vinifera leaves; b) grinding and sieving the freeze-dried leaves in order to obtain a powder of particles having a size of less than 500 µm; c) carrying out an ultrasound assisted extraction with water in order to obtain an aqueous extracted solution; d) collecting and filtering the aqueous extracted solution; and e) optionally, removing water from the extracted solution in order to obtain a dry extract.

The removal of water from the extracted solution can be carried out by freeze-drying, using a Lyobeta 6PL pilot freeze dryer (Telstar).

For the obtention of powders with a particle size less than 500 µm, dried leaves are first ground into fine powder using a M 20 Universal mill (IKA) and then subjected to a sieve analyzer with different stainless-steel sieves.

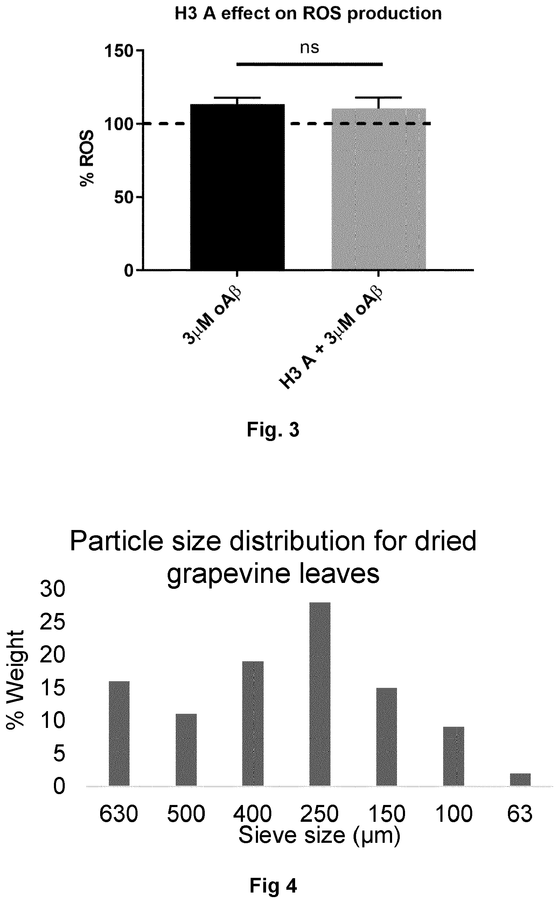

Particle size distribution is measured using a set of sieves stacked vertically with mesh sizes ranging from 630 µm to 63 µm (in particular, sieves with mesh sizes of 630 µm, 500 µm, 400 µm, 250 µm, 150 µm, 100 µm, and 63 µm) and using an electromagnetic sieve shaker (CISA, RP-03). A sample of dried leaves (typically 100 g) is vibrated through the sieves for a fixed time period (typically 10 min) and the quantity retained on each sieve is measured. The average particle size distribution of the dried leaves is shown in Fig. 4.

The terms "particle size", as used herein, is in terms of diameter irrespective of the actual particle shape. The term "diameter", as used herein, means the equivalent sphere diameter, namely the diameter of a sphere having the same diffraction pattern, when measured by laser diffraction, as the particle.

In an embodiment of the process of the invention, the ultrasound assisted extraction of step b) is carried out at a temperature from room temperature (i.e. from 20-25 °C) to 40 °C.

In another embodiment, optionally in combination with any of the embodiments provided above, the solvent is water, more particularly ultrapure water.

In the present disclosure, it is noted that when discussing the *Vitis vinifera* leaf extract, and the process for its preparation, each one of the embodiments or features defined for any of the mentioned aspects can be considered applicable to each one of the other aspects, whether or not they are explicitly discussed in the context of that other aspect. Thus, for example, when defining embodiments of the *Vitis vinifera* leaf extract *per se*, such embodiments also refer to the process for the preparation of the *Vitis vinifera* leaf extract.

A *Vitis vinifera* leaf extract obtainable by the process as defined above also forms part of the invention. Particularly, in an embodiment, the quercetin derivative is selected from the group consisting of quercetin 3-O-rutinoside, quercetin 3-O-glucoside, quercetin 3-O-galactoside, quercetin 3-O-glucuronide, and mixtures thereof; and the kaempferol derivative is selected from the group consisting of kaempferol 3-O-galactoside, kaempferol 3-O-glucoside, kaempferol 3-O-glucuronide, and mixtures thereof. In a more particular embodiment, the quercetin derivative is a mixture of 3-O-rutinoside, quercetin 3-O-glucoside, quercetin 3-O-galactoside, and quercetin 3-O-glucuronide; and the kaempferol derivative is a mixture of kaempferol 3-O-galactoside, kaempferol 3-O-glucoside, and kaempferol 3-O-glucuronide.

Throughout the description and claims the word "comprise" and variations of the word, are not intended to exclude other technical features, additives, components, or steps. Furthermore, the word "comprise" encompasses the case of "consisting of".

The following examples and drawings are provided by way of illustration, and they are not intended to be limiting of the present invention. Furthermore, the present invention covers all possible combinations of particular and preferred embodiments described herein.

### Examples

### Example 1 and Comparative Example 1- Extracts

Grape leaves were first freeze-dried and then, the samples were ground and sieved in order to obtain a powder of particles having a size of less than 500 µm.

Two different extraction technologies were used:

### Comparative Example 1.- Accelerated Solvent Extraction (ASE)

Equipment: Thermo Scientific Dionex ASE 350 Accelerated Solvent Extractor.

Extraction: 22 mL cells were used for the extraction. The assays were performed at 1600 psi for four cycles of 5 min each. For the extraction, the dried leaves (1 g) were loaded and compacted in the cell and subjected to extraction with ultrapure water. The extracted solution was collected and filtered.

An extract H3 A having a quercetin derivatives/kaempferol derivatives ratio of 29.3 was obtained.

### Example 1.- Ultrasound Assisted Extraction (UAE)

Equipment: Sonics Materials VCX 750 Ultrasonic Microprocessor.

Extraction: 2 g of dried leaves were placed in a volumetric flask (250 mL) made up to 100 mL with ultrapure water. Extraction conditions applied were: 6 mm diameter sonotrodo tip, 15 minutes extraction time, 30% amplitude and cycle 0.7. Maximum temperature was set at 40 °C. The extracted solution was collected and filtered. Finally, a dry extract can be obtained by removing the solvent by freeze-drying.

The process was carried out nine times for the extract named H1 US, having a quercetin derivatives/kaempferol derivatives average ratio of 4.7 and eight times for the extract H2 US, having a quercetin derivatives/kaempferol derivatives ratio of 4.5.

### Analysis of polyphenolic content of grape leaf extracts

Polyphenolic quantitative analysis of all the extracts was carried out using high-performance liquid chromatography with diode-array detection (HPLC/DAD) using an Agilent 1260 Infinity II Prime LC System and Ultra-Performance Liquid Chromatography with mass spectrometry detector (UPLC-MS/MS) using an Acquity UPLC H-Class-tandem triple-quadrupole mass spectrometer (TQD) (Waters).

A Phenomenex Luna C18(2) column (250 mm x 4.6 mm, 5 µm) was used with the following settings: column temperature 25 °C, injection volume 20 µL, and mobile phase containing 0.1% trifluoroacetic acid (TFA) in gradient combination with acetonitrile (ACN) at a flow rate of 0.7 mL/min.

Several polyphenols reference compounds were used as external standards for the polyphenol identification and quantification.

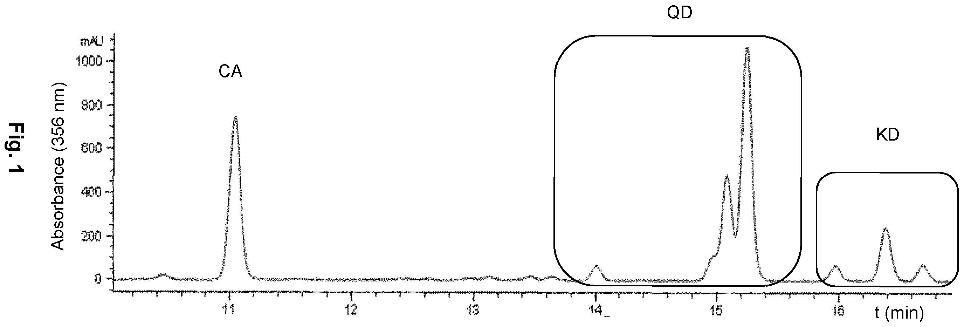

Caftaric acid, quercetin derivatives, and kaempferol derivatives shown in Table 1 below were the main polyphenols in all the grape leaf extracts obtained by different extraction methods (see, for example, Fig. 1).

**Table 1**

| Hydroxycinnamic acids | Caftaric acid |
|---|---|
| Quercetin derivatives | Quercetin 3-O-rutinoside |
| | Quercetin 3-O-glucoside |
| | Quercetin 3-O-galactoside |
| | Quercetin 3-O-glucuronide |
| Kaempferol derivatives | Kaempferol 3-O-galactoside |
| | Kaempferol 3-O-glucoside |
| | Kaempferol 3-O-glucuronide |

A detailed composition of the extracts is provided in Table 2 below.

The H1 US Concentrated extract corresponds to a dry extract obtained by freeze-drying the H1 US extract and then dissolving it in a smaller quantity of water. The results show that these processes do not affect to the Quercetin derivatives / Kaempferol derivatives ratio.

**Table 2**

| | H1 US Concentrated extract | H1 US | H2 US | H3 A |
|---|---|---|---|---|
| Caftaric acid | | | | |
| mg/L | 3350.8 | 151.2 | 114.3 | 154.6 |
| sd | - | 8.0 | 10.7 | 1.9 |
| mg/g leaf | | 6.7 | 5.1 | 7.0 |
| sd | - | 0.3 | 0.4 | 0.1 |
| Quercetin 3-O-rutinoside | | | | |
| mg/L | 167.9 | 7.3 | 8.2 | 1.4 |
| sd | - | 1.3 | 2.6 | 0.1 |
| mg/g leaf | - | 0.3 | 0.4 | 0.1 |
| sd | - | 0.1 | 0.1 | 0.0 |
| Quercetin 3-O-glucoside + Quercetin 3-O-galactoside | | | | |
| mg/L | 693.1 | 31.1 | 38.2 | 9.5 |
| sd | - | 12.4 | 13.5 | 1.0 |
| mg/g leaf | - | 1.4 | 1.7 | 0.4 |
| sd | - | 0.5 | 0.5 | 0.0 |
| Quercetin 3-O-glucuronide | | | | |
| mg/L | 3203.7 | 126.7 | 134.9 | 119.2 |
| sd | - | 17.2 | 16.8 | 13.0 |
| mg/g leaf | - | 5.6 | 6.0 | 5.4 |
| sd | - | 0.8 | 0.8 | 0.6 |
| Quercetin derivatives | | | | |
| mg/L | 4064.8 | 165.1 | 181.3 | 130.1 |
| sd | - | | | |
| mg/g leaf | - | 7.3 | 8.0 | 5.9 |
| sd | - | | | |
| Kaempferol 3-O-galactoside | | | | |
| mg/L | 83.2 | 3.9 | 5.1 | 0.0 |
| sd | - | 1.9 | 2.5 | 0.0 |
| mg/g leaf | - | 0.2 | 0.2 | 0.0 |
| sd | - | 0.1 | 0.1 | 0.0 |
| Kaempferol 3-O-glucoside | | | | |
| mg/L | 535.1 | 21.1 | 25.1 | 3.1 |
| sd | - | 4.6 | 5.7 | 0.2 |
| mg/g leaf | - | 0.9 | 1.1 | 0.1 |
| sd | - | 0.2 | 0.2 | 0.0 |
| Kaempferol 3-O-glucuronide | | | | |
| mg/L | 277.2 | 10.1 | 9.7 | 1.4 |
| sd | - | 2.7 | 1.4 | 0.1 |
| mg/g leaf | - | 0.4 | 0.4 | 0.1 |
| sd | - | 0.1 | 0.1 | 0.0 |
| Kaempferol derivatives | | | | |
| mg/L | 895.5 | 35.1 | 39.9 | 4.4 |
| sd | - | | | 0.3 |
| mg/g leaf | - | 1.5 | 1.8 | 0.2 |
| sd | - | | | |
| Quercetin derivatives / Kaempferol derivatives | | | | |
| in extract | 4.5 | **4.7** | **4.5** | 29.3 |
| in leaf | - | **4.7** | **4.6** | 29.3 |

### Example 2 - In vitro evaluation of the neuroprotective effect

The following *in vitro* assay, where the oxidative stress induced by oligomers of the amyloid β1-42 (oAβ) peptide in primary cultures of cortical neurons was measured, was used as a model of the Alzheimer's disease.

Amyloid β1-42 (Aβ) peptide is also known as amyloid-beta protein 42 (PubChem reference CID 57339251 (accessed December 14, 2022); CAS reference 107761-42-2).

### Primary cultures of cortical neurons

Neurons were obtained from E18/E19 Sprague-Dawley rat embryos as previously described (Alberdi *et al*., 2018), with modifications. Cortical lobes were digested in 0.2% trypsin and 0.02% deoxyribonuclease I in Hanks' balanced salts no calcium, no magnesium (HBSS) (Sigma-Aldrich, MO, USA) for 5 min at 37 °C. The enzymatic digestion was stopped with neurobasal medium supplemented with 2% B27 (Gibco, MA, USA), 1% penicillin-streptomycin (Lonza, Switzerland) and 0.5 mM L-glutamine (referred to as B27 neurobasal from now on), plus 10% fetal bovine serum (FBS) (Gibco, MA, USA). After incubating 10 min at 37 °C, cells were centrifuged for 5 min at 200 g, and the resultant pellet was resuspended in B27 neurobasal plus 10% FBS. Cells were mechanically dissociated by trituration (20 strokes with 21, 23 and 25-gauge needles), filtered through a 41 µm nylon mesh and seeded onto poly-D-lysine-coated (PDL) (Sigma-Aldrich, MO, USA) plates as needed. After one day, the medium was replaced by serum-free B27 neurobasal. Cultures were maintained at 37 °C and 5% CO2 for eight to nine days in vitro (DIV) before use.

### Preparation of soluble oligomeric Aβ1-42 (oAβ)

Synthetic Aβ oligomers were prepared as previously reported (Alberdi *et al*., 2018). Briefly, Aβ₁₋₄₂ (H-1368.0025, Bachem, Switzerland) was dissolved in hexafluoroisopropanol (HFIP) (Sigma-Aldrich, MO, USA) to an initial concentration of 1 mM. It was distributed into aliquots and HFIP was removed under vacuum in a speed vac system before the peptide film was stored dry at -80 °C. For the aggregation protocol, the peptide was resuspended in anhydrous dimethylsulfoxide (DMSO) (Invitrogen, MA, USA) to a concentration of 5 mM. Hams F-12 pH 7.4 (Biowest, France) was added to reach the final concentration of 100 µM. Oligomers were obtained after incubating at 4 °C for 24 h.

### Measurement of Reactive Oxygen Species (ROS)

The following assay was carried out with any one of the two extracts obtained in Example 1 (H1 US and H2 US) and with the extract obtained in Comparative Example 1 (H3 A).

Primary neurons were treated with 3 µM oAβ for 1 h and the corresponding extracts (1:1000 dilution in culture media). The extract was added 1 h before oAβ. Probes for ROS detection were added in the last 30 min of incubation. 5,6-chloromethyl-2'7dichlorodihydrofluorescein diacetate acetyl ester (CM-H2DCFDA) (30 µM, Invitrogen, MA, USA) was used to assess intracellular ROS levels. 4% NucBlueTM was also added for normalization. After the incubation, cultures were washed twice with 1X PBS and fluorescence was measured using a microplate reader Synergy HT and the software KC4. Excitation/emission ranges were 490/520 nm for CM-H2DCFDA and 360/460 nm for NucBlueTM.

### Results

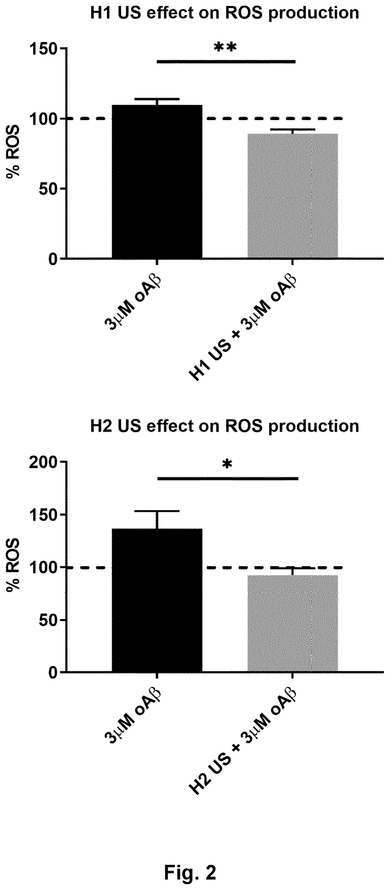

Grape leaf extracts obtained by UAE, such as H1 US and H2 US extracts, showed a neuroprotective effect against oxidative stress (see Fig. 2) represented by the significant reduction of free radicals (ROS) produced in the presence of oligomers of the amyloid β1-42 (oAβ) peptide.

On the contrary, grape leaf extracts obtained by ASE, namely the H3 A extract, did not show neuroprotection against oxidative stress produced by oAβ. (see Fig. 3)

Therefore, grape leaf extracts obtained by the process of the present disclosure (i.e., by UAE extraction, that is, a grape leaf extract with a quercetin derivatives/kaempferol derivatives ratio from 4.2 to 5.0) provides a better neuroprotective effect than a grape leaf extract obtained by ASE extraction (wherein the quercetin derivatives/Kaempferol derivatives ratio is significantly higher than the range defined for the UAE extract).

### Citation List

1. Alberdi E, et al. "Mangiferin and morin attenuate oxidative stress, mitochondrial dysfunction, and neurocytotoxicity, induced by Amyloid Beta Oligomers". Oxid Med Cell Longer 2018, vol. 2018:2856063; doi: 10.1155/2018/2856063

## Claims

1. A *Vitis vinifera* leaf extract comprising caftaric acid, a quercetin derivative, and a kaempferol derivative, **characterized in that** the quercetin derivative/kaempferol derivative weight ratio is from 4.2 to 5.0.

2. The extract according to claim 1, wherein caftaric acid is in an amount from 30 wt% to 45 wt% with respect to the total amount of caftaric acid, quercetin derivative, and kaempferol derivative.

3. The extract according to claims 1 or 2, wherein the extract is a dry extract and the amount of caftaric acid is from 38 wt% to 46 wt% with respect to the total amount of dry extract.

4. The extract according to claims 1 to 3, wherein the quercetin derivative is selected from the group consisting of quercetin 3-O-rutinoside, quercetin 3-O-glucoside, quercetin 3-O-galactoside, quercetin 3-O-glucuronide, and a mixture thereof; and the kaempferol derivative is selected from the group consisting of kaempferol 3-O-galactoside, kaempferol 3-O-glucoside, kaempferol 3-O-glucuronide, and a mixture thereof.

5. The extract according to claim 4, wherein the quercetin derivative is a mixture of 3-O-rutinoside, quercetin 3-O-glucoside, quercetin 3-O-galactoside, and quercetin 3-O-glucuronide; and the kaempferol derivative is a mixture of kaempferol 3-O-galactoside, kaempferol 3-O-glucoside, and kaempferol 3-O-glucuronide.

6. A pharmaceutical composition comprising a therapeutically effective amount of the extract defined in any one of claims 1 to 5 and at least one pharmaceutically acceptable excipient or carrier.

7. The pharmaceutical composition according to claim 6, wherein the *Vitis vinifera* leaf extract is in an amount from 0.75 µg to 3.0 µg of dry extract.

8. The extract according to any one of claims 1 to 5, or the pharmaceutical composition according to claims 6 or 7, for use in medicine.

9. The extract according to any one of claims 1 to 5, or the pharmaceutical composition according to claim 6 or 7, for use in the prevention or in the treatment of a neurodegenerative disease.

10. The extract for use or the pharmaceutical composition for use of claim 9, wherein the neurodegenerative disease is the Alzheimer's disease.

11. A process for the preparation of the *Vitis vinifera* leaf extract as defined in any one of claims 1 to 3, the process comprising:
a) freeze-drying *Vitis vinifera* leaves,
b) grinding and sieving the freeze-dried leaves in order to obtain a powder of particles having a size of less than 500 µm;
c) carrying out an ultrasound assisted extraction with water in order to obtain an aqueous extracted solution;
d) collecting and filtering the aqueous extracted solution; and
e) optionally, removing water from the aqueous extracted solution in order to obtain a dry extract.

12. The process of claim 11, wherein ultrasound assisted extraction of step b) is carried out at a temperature from room temperature to 40 °C.

13. The process of claims 11 or 12, wherein the solvent is ultrapure water.

14. The process of any one of claims 11 to 13, wherein removal of water is carried out by freeze-drying.

15. A *Vitis vinifera* leaf extract obtainable by the process of any one of claims 11 to 14, wherein the extract comprises caftaric acid, a quercetin derivative, and a kaempferol derivative, and the quercetin derivative/kaempferol derivative weight ratio is from 4.2 to 5.0.
